# FASCICULE DE BREVET EUROPEEN

(11) **EP 0 706 512 B1**
(45) Date de publication et mention de la délivrance du brevet: **08.04.1998**
(21) Numéro de dépôt: 94921005.8
(22) Date de dépôt: 30.06.1994
(51) Int. Cl.: C07C 317/44, C07C 323/60, C07D 213/56, C07D 213/40, A61K 31/16, A61K 31/44

(54) **DERIVES D'ACETAMIDE ET LEUR UTILISATION COMME MODIFICATEURS DU COMPORTEMENT DE PRISE ALIMENTAIRE**
ACETAMIDDERIVATE UND IHRE VERWENDUNG ALS MODIFIZIERUNGSMITTEL DES VERHALTENS DER VERDAUUNG
ACETAMIDE DERIVATIVES AND THEIR USE FOR MODIFYING FEEDING BEHAVIOUR MODIFIERS

(30) Priorité: 30.06.1993 FR 9308008
(43) Date de publication de la demande: 17.04.1996
(73) Titulaire: LABORATOIRE L. LAFON, 94701 Maisons Alfort (FR)
(72) Inventeur: LAURENT, Philippe, F-69600 Oullins (FR)
(74) Mandataire: Le Guen, Gérard
(86) Numéro de dépôt international: FR9400803
(87) Numéro de publication internationale: WO9501333

(56) Documents cités:
- FR-A- 2 385 693
- FR-A- 2 528 038
- FR-A- 2 602 768

## Description

La présente invention concerne des dérivés d'acétamide utilisables en thérapeutique comme modificateurs du comportement de prise alimentaire.

On connaît déjà différents dérivés d'acétamide utilisables en thérapeutique pour leur effet stimulant sur le système nerveux central. On peut citer notamment l'adrafinil ou acide benzhydrylsulfinyl acétohydroxamique (décrit dans FR-A-2 326 181) et le modafinil (décrit dans FR-A-2 385 693) et d'autres composés décrits dans FR-A-2 528 038, FR-A-2 601 673, FR-A-2 602 768 et FR-A-2 606 815.

La présente invention vise à fournir de nouveaux dérivés d'acétamide qui se caractérisent par un effet modificateur ou régulateur du comportement de prise alimentaire chez l'homme et les animaux.

La présente demande a ainsi pour objet des composés de formule : 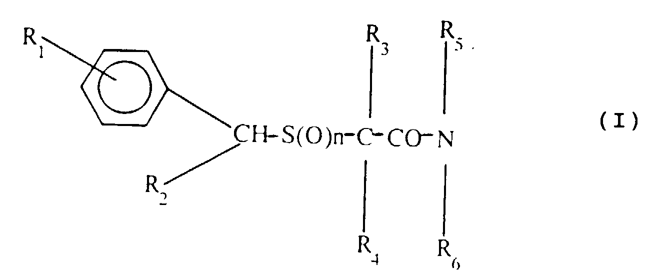 dans laquelle :
R₁ est choisi parmi H et 3-chloro,
R₂ est choisi parmi un groupe phényle et un groupe pyridyle,
R₃ et R₄ sont choisis indépendamment l'un de l'autre parmi H et méthyle,
R₅ et R₆ sont choisis indépendamment l'un de l'autre parmi H et un groupe pyridyl-méthyle ou représentent tous les deux un groupe éthyle,
n = 0 ou 1,
R₁, R₃, R₄, R₅ et R₆ n'étant pas simultanément H quand R₂ = phényle,
et les sels des composés à groupe pyridyle avec des acides pharmaceutiquement acceptables.

D'une manière générale, les composés de formule I peuvent être obtenus selon des méthodes connues. Ainsi les composés de formule I dans lesquels n = 1 peuvent être obtenus par oxydation, en particulier par H₂O₂, d'un composé de formule I dans laquelle n = 0, c'est-à-dire d'un thioacétamide de formule :

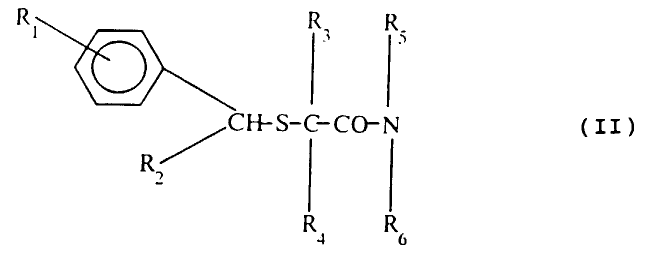

Les thioacétamides de formule II peuvent être obtenus notamment par réaction d'un dérivé réactif d'un acide de formule : 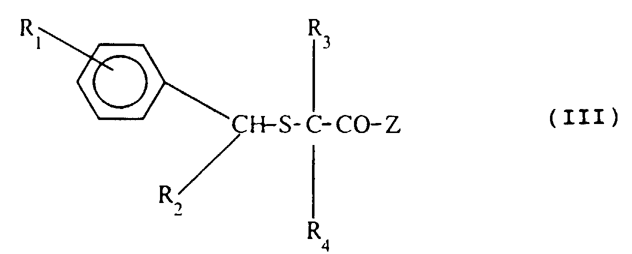 dans laquelle Z est un groupe réactif, notamment Cl ou OCH₃
avec une amine de formule :

HNR₅R₆ (IV)

En variante, certains composés peuvent être obtenus par réaction d'un acide de formule : 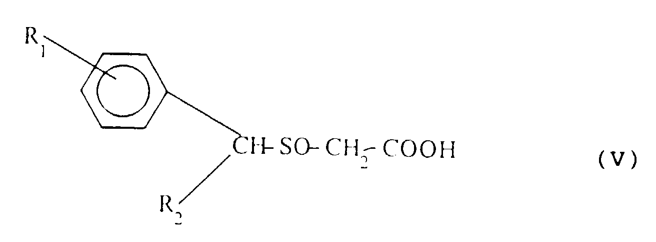 ou d'un de ses dérivés réactifs,
avec une amine de formule III.

Les composés selon l'invention peuvent être utilisés en thérapeutique comme modificateurs ou régulateurs du comportement de prise alimentaire chez l'homme et les animaux.

La présente invention a donc également pour objet des compositions thérapeutiques comprenant une quantité efficace d'un composé de formule I ou des sels des composés de formule à groupe pyridine avec des acides pharmaceutiquement acceptables.

Les compositions thérapeutiques selon l'invention peuvent être administrées notamment par voie orale.

Elles peuvent être sous la forme de préparations solides ou semi-solides. Comme exemple, on peut citer les comprimés, les gélules, ainsi que les formes-retard.

Dans ces compositions, le principe actif est généralement mélangé avec un ou plusieurs excipients pharmaceutiquement acceptables habituels bien connus de l'homme de l'art.

Ces compositions permettent en particulier de retarder la prise alimentaire et peuvent donc constituer un aide précieuse pour la régulation du poids, notamment chez des individus qui suivent un régime alimentaire.

Les effets sur le comportement de prise alimentaire ont été mis en évidence à l'aide d'un test chez la souris selon une méthode décrite par Ladurelle et col. (Fundam. Clin. Pharmacol., 5, 481).

Les exemples suivants illustrent la préparation des composés de formule I.

### EXEMPLE 1

### Préparation du m-chlorobenzhydrylsulfinylacétamide (CRL 41096)

**a) Préparation du m-chlorobenzhydrol**
   Dans un tricol on place : 4,86 g (0,2 Atg) de magnésium recouvert d'un peu d'éther anhydre, ajoute un cristal d'iode et quelques gouttes de bromobenzène de façon à faire démarrer la réaction; puis on additionne goutte à goutte une solution de 32 g (0,204 mole) de bromobenzène dans 200 ml d'éther. On maintient ensuite le reflux au bain-marie pendant environ 1 heure puis refroidit et ajoute goutte à goutte, à froid, une solution de 28,1 g (0,2 mole) de m-chlorobenzaldéhyde dans un peu d'éther. Le complexe ainsi obtenu est filtré, puis repris à l'éther et hydrolysé par une solution d'eau + HCl. La phase éthérée est alors lavée à l'eau, séchée sur Na₂SO₄, évaporée.
   On recueille 28,14 g d'une huile jaune : le m-chlorobenzhydrol (Rdt. = 65 %).
**b) Préparation de l'acide m-chlorobenzhydrylthioacétique**
   Dans un tricol on place : 12,86 g (0,156 mole) de thiourée dans 65 ml d'HBr à 48 % et 13 ml d'eau, on porte l'ensemble à 60° C et ajoute en une seule fois 28,4 g (0,13 mole) de m-chlorobenzhydrol. On élève la température à 95° C puis laisse refroidir : le bromhydrate de thiouronium précipite; on essore celui-ci et lave à l'eau.
   Le sel ainsi obtenu est repris avec 39 ml de lessive de soude 10 N (0,39 mole), l'ensemble porté à 70° C et additionné goutte à goutte d'une solution de 13,51 g (0,143 mole) d'acide chloroacétique dans un peu d'eau. On porte alors au reflux et maintient celui-ci 1/2 heure puis refroidit : le sel de sodium de l'acide précipite.
   On acidifie directement avec HCl concentré, extrait l'huile ainsi obtenue à l'éther, lave avec une solution de soude diluée, filtre sur charbon, acidifie de nouveau avec HCl concentré, essore le précipité ainsi obtenu et lave à l'eau. On obtient après recristallisation dans le cyclohexane : 29,29 g d'acide m-chlorobenzhydrylthioacétique (F._{inst.} = 97° C; Rdt. = 77 %).
**c) Préparation du chlorure de m-chlorobenzhydrylthioacétique**
   On dissout 29,25 g (0,1 mole) de l'acide obtenu en b) dans 150 ml de chlorure de thionyle. On maintient le reflux 1 heure, puis refroidit, évapore le benzène et le chlorure de thionyle en excès.
   On recueille une huile jaune (Rdt. ≠≠ 100 %).
**d) Préparation du m-chlorobenzhydrylthioacétamide**
   Dans un tricol on place 50 ml de NH₃ dissous dans 60 ml d'eau, refroidit et ajoute goutte à goutte le chlorure d'acide précédemment obtenu ( ≃ 0,1 mole) dissous dans environ 125 ml de chlorure de méthylène. On laisse le mélange en contact une nuit à température ambiante puis lave avec une solution de soude diluée puis à l'eau, sèche sur Na₂SO₄, évapore. On recueille une huile jaune orangée qui cristallise dans l'eau.
   On obtient 27,5 g de m-chlorobenzhydrylthioacétamide (F._{inst.} = 56° C ; Rdt = 94 %).
**e) Préparation du m-chlorobenzhydrylsulfinylacétamide**
   On dissout dans un ballon 26,23 g (0,09 mole) du produit obtenu en d) dans 90 ml d'acide acétique et ajoute 9,5 ml d'H₂O₂ (≃ 110 vol.). La température monte jusqu'à 45° C puis redescend. On suit la disparition du sulfure en chromatographie puis ajoute H₂O et neutralise l'acide acétique par addition de bicarbonate de sodium, essore le précipité ainsi obtenu et lave à l'eau. On recueille après recristallisation dans l'éthanol : 14 g de m-chlorobenzhydrylsulfinylacétamide (F._{inst.} = 170° C; Rdt = 50 %).
   Le produit est une poudre blanche, insoluble dans l'éther, l'acétate d'éthyle, légèrement soluble dans les alcools. Sa solubilité dans l'eau est inférieure à 0,1 %.

### EXEMPLE 2

### Préparation du N-diéthyl benzhydryl sulfinyl acétamide (CRL 40488)

a) Préparation du N-diéthyl benzhydryl thioacétamide
   Dans un tricol muni d'un réfrigérant et d'une ampoule de coulée on place 50 ml (0,5 mole) de diéthylamine dans environ 50 ml d'éther, et l'on ajoute goutte à goutte 27,2 g (0,998 mole) du chlorure de benzhydryl thioacétyle en solution dans environ 100 ml de benzène. Une fois l'addition terminée, le mélange réactionnel est additionné d'eau. On lave la phase organique avec une solution de soude diluée d'acide chlorhydrique dilué, puis à l'eau, sèche sur Na₂SO₄, évapore le solvant.
**b) Préparation du N-diéthyl benzhydryl sulfinyl acétamide**
   On met dans un ballon 24,42 g (0,078 mole) de l'acétamide brut précédemment obtenu, ajoute 80 ml d'acide acétique et 8 ml de H₂O₂ (environ 100 vol.). Le mélange est laissé en contact une nuit à température ambiante. On évapore l'acide acétique, reprend l'huile obtenue en chlorure de méthylène, lave avec une solution de soude diluée, d'acide chlorhydrique dilué, puis à l'eau, sèche sur Na₂SO₄, évapore le solvant, reprend à l'éther isopropylique, on cristallise ainsi le produit. On recristallise dans le benzène et l'éther isopropylique et on recueille 16,6 g de N-diéthyl benzhydryl sulfinyl acétamide (Rdt. = 65 %. F._{inst.} = 100-101° C).
   Le produit est une poudre blanche, légèrement soluble dans l'éther, l'éther isopropylique, soluble dans les alcools, le chlorure de méthylène. Sa solubilité dans l'eau est inférieure à 0,1 %.

### EXEMPLE 3

### Préparation du chlorhydrate de l'α-(4-pyridyl) benzylsulfinylacétamide (CRL 41890)

**a) Préparation du chlorhydrate du chlorure d'α(4-pyridyl) benzyle**
   Dans une solution de 27,75 g (0,15 mole) d'alcool (4-pyridyl)benzylique et 200 ml de chloroforme, on coule goutte à goutte à l'ambiante, une solution de 15,25 ml (0,2 mole) de SOCl₂ et 50 ml de CH₂Cl₂.
   Après trois heures au reflux, on évapore à sec sous vide, reprend le résidu à l'acétate d'éthyle, essore, sèche et obtient le chlorhydrate recherché (F. = 152-154° C) avec un rendement de 96 %.
**b) Préparation de l'acide α-(4-pyridyl)benzylthioacétique**
   On chauffe à 40° C une solution de 10,2 g (0,13 mole) de thiourée dans 100 ml d'eau, ajoute 28,8 g (0,12 mole) du chlorhydrate obtenu en a) et chauffe 10 minutes à reflux. On ajoute à 50° C une solution de 24 g (0,6 mole) de soude dans 150 ml d'eau et chauffe 10 minutes à reflux.
   On refroidit à 50° C et coule goutte à goutte une solution de 12,6 g (0,132 mole) d'acide chloroacétique, 10 g de CO₃Na₂ et 100 ml d'eau. On chauffe ensuite 1 heure à reflux, refroidit, extrait 2 fois avec 100 ml d'éther, filtre sur charbon, précipite avec HCl concentré, essore et sèche.
   L'acide (F. = 142° C) est ainsi obtenu avec un rendement de 56 %.
**c) Préparation de l'α(4-pyridyl) benzylthioacétamide**
   On chauffe 8 heures à reflux une solution de 19,2 g (0,075 mole) de l'acide obtenu en b) dans 300 ml de méthanol et 10 ml de H₂SO₄ concentré, évapore sous vide le méthanol, reprend le résidu avec 500 ml d'eau froide, précipité avec CO₃Na₂, extrait à l'éther, lave à l'eau, sèche, évapore sous vide.
   L'ester méthylique est dissout dans 100 ml de méthanol et on ajoute 50 ml d'ammoniaque à 28 %, après 48 heures en contact, on évapore à sec sous vide, reprend avec 100 ml d'eau, essore et sèche.
   On obtient l'amide (F. = 126° C) avec un rendement de 84 %.
   La base en solution dans le méthanol est transformée quantitativement en chlorhydrate (F. = 192-194° C) par addition d'isopropanol chlorhydrique.
**d) Préparation du 4-chlorhydrate de l'α (4-pyridyl) benzylsulfinylacétamide**
   9,3 g (0,03 mole) du sulfure obtenu en c) en solution dans 30 ml d'acide acétique, sont oxydés par addition de 3 ml d'eau oxygénée à 110 volumes. On agite 1 heure à 45° C environ et 2 heures à 25° C, évapore à sec sous vide, reprend à l'éthanol, essore et recristallise dans le méthanol.
   On obtient le produit avec un rendement de 76 %.
   Il se présente sous la forme de cristaux blancs.
   Il est soluble dans l'eau, le méthanol, insoluble dans l'éthanol, l'éther, l'acétone, etc ...
   Il fond avec décomposition à 182-183° C.

### EXEMPLE 4

### Préparation du 2-(benzhydryl sulfinyl)-propionamide (CRL 41163)

**a) Préparation de l'acide 2-(benzhydrylthio)-propionique**
   Dans un tricol on place 7,6 g (0,1 mole) de thiourée dans 50 ml d'eau et ajoute en une seule fois à 50-60° C 18 ml (0,1 mole) de chlorodiphénylméthane. On chauffe alors jusqu'à reflux et maintient 1/4 d'heure à ébullition. La solution devient limpide. On refroidit ensuite et ajoute de nouveau vers 60° C goutte à goutte, une solution de 16 g (0,4 mole) de soude dans 25 ml d'eau. On porte alors au reflux et maintient celui-ci 1/2 h - 3/4 h, refroidit et ajoute goutte à goutte vers 65 - 70° C une solution d'environ 0,14 mole de 2-bromopropionate de potassium (obtenue en neutralisant dans 100 ml d'eau 21,5 g (soit 13 ml) d'acide α-bromopropionique par 14,2 g de bicarbonate de potassium.
   L'ensemble est ensuite porté à reflux 1/2 heure puis on refroidit, acidifie directement dans le milieu réactionnel avec HCl concentré, essore le précipité ainsi obtenu et lave à l'eau.
   L'acide est purifié en le redissolvant à froid dans une solution de soude diluée, l'insoluble extrait au chlorure de méthylène, la phase alcaline filtrée sur charbon puis acidifiée. L'acide ainsi obtenu est essoré et lavé à l'eau.
   On obtient 20 g d'acide 2-(benzhydrylthio)-propionique.
   (F._{inst.} = 148° C ; Rdt. = 75 %).
**b) Préparation du chlorure de l'acide 2-(benzhydrylthio)-propionique**
   Dans un tricol on place 19,04 g (0,07 mole) de l'acide obtenu en a) dans 120 ml de benzène, porte à reflux (solution) et ajoute goutte à goutte 18 ml de chlorure de thionyle. Lorsque l'addition est terminée, on poursuit une heure le reflux puis refroidit, évapore le benzène ainsi que l'excès de chlorure de thionyle. On recueille une huile orrangée limpide (Rdt ≃ 100 %).
**c) Préparation du 2-(benzhydrylthio)-propionamide**
   On dissout 35 ml d'ammoniaque dans 40 ml d'eau, refroidit et additionne goutte à goutte le chlorure d'acide précédemment obtenu (0,07 mole) dissous dans un peu de chlorure de méthylène. On laisse une nuit en contact puis décante la phase organique, avec une solution de soude siluée de façon à éliminer les traces d'acide, lave à l'eau jusqu'à pH neutre, sèche sur Na₂SO₄, évapore, reprend le précipité ainsi obtenu à l'éther de pétrole.
   On obtient ainsi après recristallisation dans l'éthanol : 12,82 g de (benzylthio)-2 propionamide.
   F._{inst.} = 110° C ; Rdt. = 68 %).
**d) Préparation du 2-(benzhydryl sulfinyl) propionamide**
   Dans un ballon, on place 13,55 g (0,05 mole) de 2-(benzhydrylthio)-propionamide, ajoute 50 ml d'acide acétique et 6,5 ml de H₂O₂ (≃ 110 v). La température monte jusqu'à 40° C puis redescend; lorsque le totalité du sulfure a disparu, on évapore l'acide acétique, reprend à l'eau et au bicarbonate de sodium, essore le précipité ainsi obtenu et lave à l'eau.
   On recueille après cristallisation dans l'acétate d'éthyle : 8 g de 2-(benzhydrylsulfinyl)-propionamide. (F._{inst.} = 141° C ; Rdt = 60 %).
   Le produit est une poudre blanche, insoluble dans l'éther, très légèrement soluble dans l'acétate d'éthyle, l'acétone, les alcools. Sa solubilité dans l'eau est inférieure à 0,1 %.

### EXEMPLE 5

### Préparation du 2-(benzhydryl sulfinyl)-2 isobutyramide (CRL 41412)

**a) Préparation de l'acide 2-(benzhydrylthio)-isobutyrique**
   Dans une solution de 6 g (0,26 Atg) de sodium dans 500 ml d'éthanol, on ajoute 40 g (0,2 mole) de diphénylméthane thiol, puis coule goutte à goutte en 1 heure, 48,75 g (0,25 mole) de bromo-2 isobutyrate d'éthyle. On maintient le reflux environ 2 heures, puis évapore l'éthanol, reprend à l'éther, lave à l'eau trois fois, sèche, évapore.
   On recueille 59,6 g de 2-(benzhydrylthio)-isobutyrate d'éthyle. L'ester précédemment obtenu est alors dissous dans 100 ml d'éthanol et l'on ajoute goutte à goutte à froid : 33,6 g (0,6 mole) de KOH dans l'alcool. On évapore ensuite l'éthanol, reprend à l'eau (solution), filtre sur C, acidifie avec HCl concentré.
   On purifie l'acide en le redissolvant dans une solution de soude diluée, et on acidifie de nouveau.
   On obtient, après recristallisation dans le cyclohexane : 28 g d'acide 2-(benzhydrylthio)-isobutyrique. (F._{inst.} = 119° C; Rdt = 50 %).
**b) Préparation du 2-(benzhydrylthio)-isobutyramide**
   25,74 g (0,09 mole) du produit précédent sont dissous dans 180 ml de benzène et l'on ajoute goutte à goutte, au reflux, 22,5 ml de chlorure de thionyle. On poursuit le reflux 1 heure, puis refroidit, évapore le benzène, reprend le chlorure d'acide ainsi obtenu avec 100 ml de chlorure de méthylène et additionne celui-ci goutte à goutte, à une solution de 200 g de glace et 100 ml d'ammoniaque.
   Après une nuit en contact, la phase organique est lavée à l'eau, séchée, évaporée; le précipité ainsi obtenu est lavé à l'éther isopropylique puis essoré.
   On recueille 15,1 g de 2-(benzhydrylthio)-isobutyramide.
   (F._{inst.} = 140° C ; Rdt = 59 %).
**c) Préparation du 2-(benzhydrylsulfinyl)-isobutyramide**
   Dans un ballon on place 14,25 g (0,05 mole) de l'amide obtenue en b), ajoute 50 ml d'acide acétique et 6 ml d'H₂O₂ ( ~ 110 vol.).
   La température s'élève à 40° C puis redescend. On évapore ensuite l'acide acétique, reprend à l'éther isopropylique, précipite ainsi et essore le 2-(benzhydrylsulfinyl)-isobutyramide.
   On obtient après recristallisation dans l'alcool isopropylique, 9 g de 2-(benzhydrylsulfinyl)-isobutyramide.
   (F._{dec.} = 117° C; Rdt = 60 %).
   Le produit est une poudre blanche, insoluble dans l'éther, légèrement soluble dans l'acétate d'éthyle, soluble dans les alcools. Sa solubilité dans l'eau est de l'ordre de 0,1 %.

### EXEMPLE 6

### Préparation du chlorhydrate du N-(2-pyridyl méthyl) benzhydrylsulfinyl acétamide (CRL 41936)

**a) Préparation du N-(2-pyridyl méthyl) benzhydrylsulfinyl acétamide**
   Dans une suspension de 15,44 g (0,06 mole) d'acide benzhydrylsulfinyl acétique dans 90 ml de chlorure de méthylène, on ajoute 6,48 g (0,06 mole soit 6,17 ml) de 2-aminométhyl pyridine et dans la solution ainsi obtenue 12,36 g (0,06 mole) de dicyclohexylcarbodiimide. On chauffe le mélange environ 3 à 4 heures à reflux, puis laisse refroidir.
   Le lendemain, on filtre le précipité obtenu, évapore le chlorure de méthylène, reprend à l'éther, lave à l'eau, extrait avec une solution d'HCl dilué, filtre l'insoluble, précipite avec une solution de NaOH concentrée et essore.
   On recueille 5 g de N-(2-pyridyl méthyl) benzhydrylsulfinylacétamide.
   F. _{pas nette} ≃ 76° C ; Rdt. = 24 %.
**b) Préparation du chlorhydrate du N-(2-pyridyl méthyl) benzhydrylsulfinyl acétamide**
   Le sel est préparé dans l'acétone par addition d'isopropanol chlorhydrique à une solution de la base précédemment obtenue. On recueille 4,2 g de chlorhydrate du N-(2-pyridyl méthyl) benzhydrylsulfinyl acétamide.
   F. _{pâteux} =108° C ; Rdt = 76 %.
   C'est une poudre beige, insoluble dans l'éther, l'acétate d'éthyle, soluble dans l'eau.

## Revendications

1. Composés de formule : 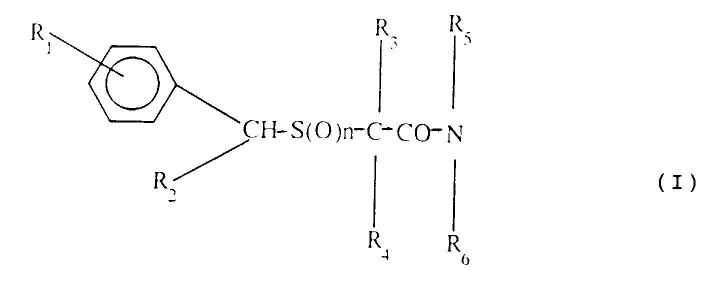 dans laquelle :
R₁ est choisi parmi H et 3-chloro,
R₂ est choisi parmi un groupe phényle et un groupe pyridyle,
R₃ et R₄ sont choisis indépendamment l'un de l'autre parmi H et méthyle,
R₅ et R₆ sont choisis indépendamment l'un de l'autre parmi H et un groupe pyridyl-méthyle ou représentent tous les deux un groupe éthyle,
n = O ou 1,
R₁, R₃, R₄, R₅ et R₆ n'étant pas simultanément H quand R₂ = phényle,
et les sels des composés à groupe pyridyle avec des acides pharmaceutiquement acceptables.

2. Composition thérapeutique comprenant une quantité efficace d'un composé selon la revendication 1.

3. Utilisation d'un composé selon la revendication 1 pour la fabrication d'un médicament modifiant le comportement de prise alimentaire.

4. Procédé de préparation d'un composé de formule I selon la revendication 1 et dans laquelle n = 1 par oxydation d'un composé de formule: 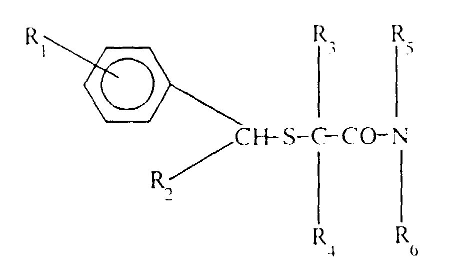 dans laquelle R₁, R₂, R₃, R₄, R₅ et R₆ ont la signification donnée à la revendication 1.

5. Procédé de préparation d'un composé de formule : 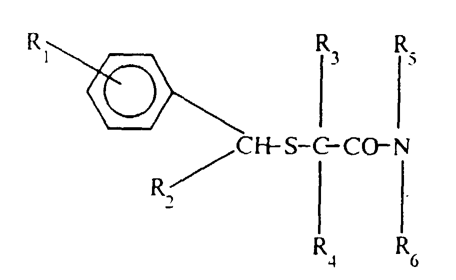 dans laquelle R₁, R₂, R₃, R₄, R₅ et R₆ ont la signification donnée à la revendication 1,
par réaction d'un dérivé réactif d'un acide de formule: 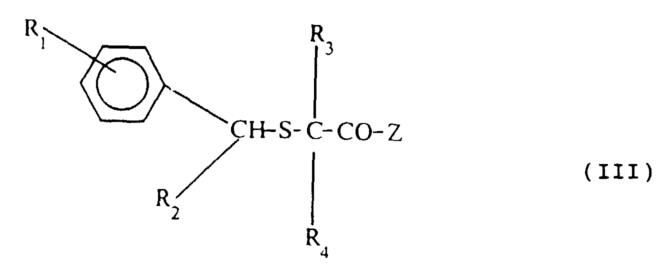 dans laquelle Z est un groupe réactif, notamment Cl ou OCH₃
avec une amine de formule :
HNR₅R₆ (IV).

## Claims

1. Compounds of the formula: 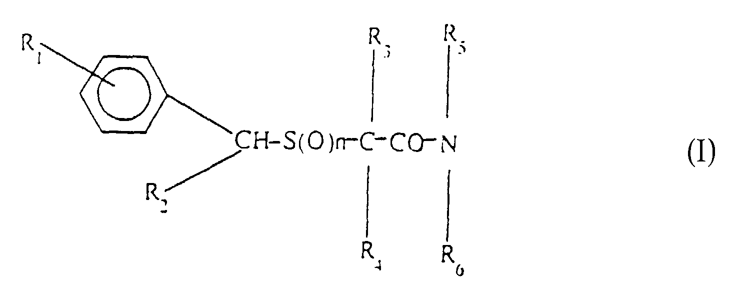 in which:
R₁ is selected from among H and 3-chloro,
R₂ is selected from a phenyl group and a pyridil group,
R₃ and R₄ are selected, independently of one another, among H and methyl,
R₅ and R₆ are selected independently of one another among H and a pyridil-methyl group in which both represent an ethyl group,
n = 0 or 1,
R_{1,}, R₃, R₄, R₅ and R₆ not being simultaneously H when R₂ = phenyl,
and the salts of the pyridil-group compounds with pharmaceutically acceptable acids.

2. Therapeutic compound comprising an effective quantity of a compound according to claim 1.

3. Use of a compound according to claim 1 for manufacturing a medicine modifying the alimentary intake behaviour.

4. Method of preparing a compound of formula I according to claim 1 and in which n = 1 by oxidation of a compound of the formula: 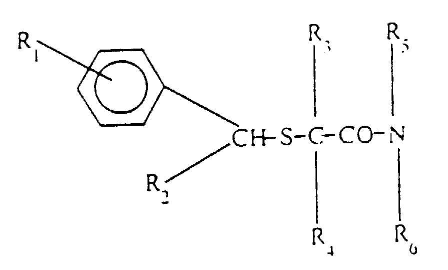 in which R₁, R₂, R₃, R₄, R₅ and R₆ have the meaning ascribed to them in claim 1.

5. Method of preparing a compound of the formula: 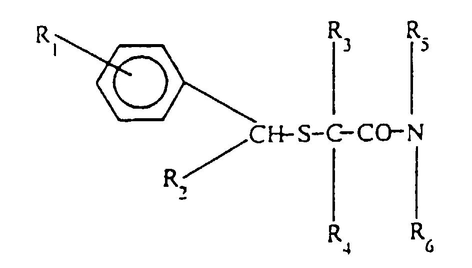 in which R₁, R₂, R₃, R₄, R₅ and R₆ have the meaning ascribed to them in claim 1,
by reaction of a reactive derivate of an acid of the formula: 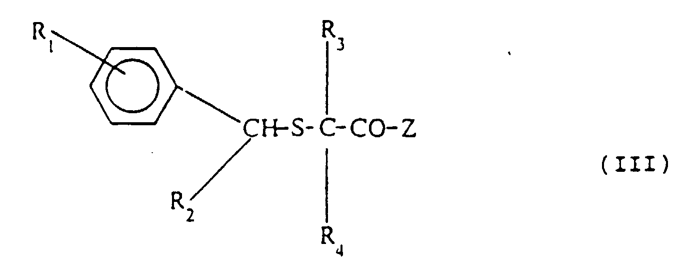 in which Z is a reactive group, particularly Cl or OH₃, with an amine of the formula:
HNR₅R₆ (IV).

## Patentansprüche

1. Verbindungen der Formel : 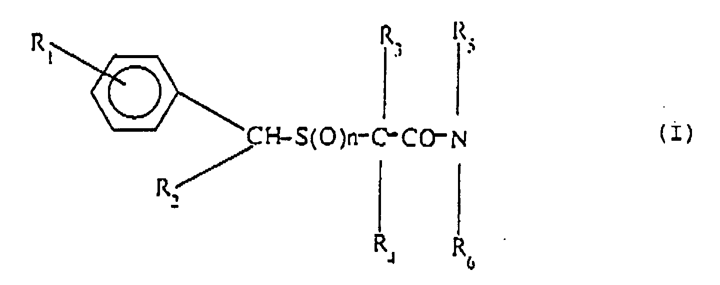 in der :
R₁ wahlweise für H und eine 3-Chlor-Gruppe steht,
R₂ wahlweise für eine Phenyl- und eine Pyridylgruppe steht,
R₃ und R₄ wahlweise und unabhängig voneinander für H und eine Methylgruppe stehen,
R₅ und R₆ wahlweise und unabhängig voneinander für H und eine Pyridylmethyl-Gruppe oder beide für eine Ethylgruppe stehen,
n = 0 oder 1,
R₁, R₃, R₄, R₅ und R₆ nicht gleichzeitig H sind, wenn R₂ = Phenyl, und die Salze Verbindungen mit Pyridyl-Gruppe mit pharmazeutisch akzeptablen Säuren.

2. Therapeutische Zusammensetzung mit einer wirksamen Menge einer Verbindung nach Anspruch 1.

3. Verwendung einer Verbindung nach Anspruch 1 für die Herstellung eines Medikaments, das das Verhalten der Verdauung modifiziert.

4. Verfahren zur Herstellung einer Verbindung der Formel I nach Anspruch 1, in der n = 1, durch Oxidation einer Verbindung der Formel: 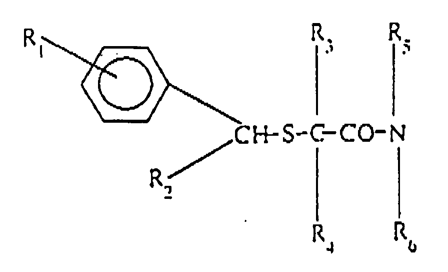 in der R₁, R₂, R₃, R₄, R₅ und R₆ die in Anspruch 1 wiedergegebene Bedeutung haben.

5. Verfahren zur Herstellung einer verbindung der Formel: 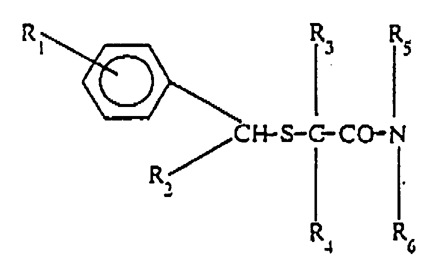 in der R₁, R₂, R₃, R₄, R₅ und R₆ die in Anspruch 1 wiedergegebene Bedeutung haben, durch Reaktion eines reaktionsfähigen Derivats einer Säure der Formel: 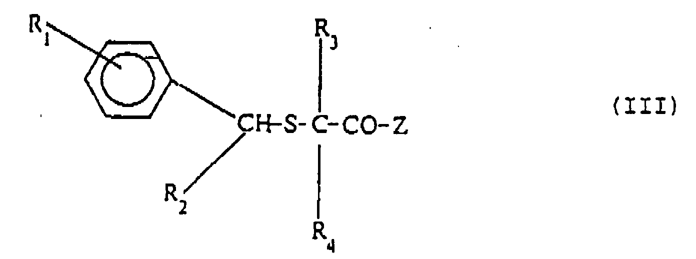 in der Z eine reaktionsfähige Gruppe, besonders Cl oder OCH₃ ist, mit einem Amin der Formel:
HNR₅R₆ (IV)
